# EUROPEAN PATENT APPLICATION

(11) **EP 2 042 194 A2**
(43) Date of publication of application: **01.04.2009**
(21) Application number: 08015392.7
(22) Date of filing: 01.05.2000
(51) Int. Cl.: A61K 39/395, A61P 35/00, C07K 16/28

(54) **Treatment of refractory human tumors with epidermal growth factor receptor antagonists**

(30) Priority: 14.05.1999 US 312284; 13.08.1999 US 374028
(62) Divisional of application: 00928671.7
(71) Applicant: IMCLONE SYSTEMS, INC., New York, NY 10014 (US)
(72) Inventor: Waksal, Harlan W., Montclair New Jersey 07042 (US)
(74) Representative: Martin, David John

(57) **Abstract**

A method of inhibiting the growth of refractory tumors that are stimulated by a ligand of epidermal growth factor in human patients, comprising treating the human patients with an effective amount of an epidermal growth factor receptor antagonist.

## Description

### BACKGROUND OF THE INVENTION

Cancer is the second leading cause of death next to heart attacks in the United States. There has been important progress in the development of new therapies in the treatment of this devastating disease. Much of the progress is due to a better understanding of cell proliferation in both normal cells and cancerous cells.

Normal cells proliferate by the highly controlled activation of growth factor receptors by their respective ligands. Examples of such receptors are the growth factor receptor tyrosine kinases.

Cancer cells also proliferate by the activation of growth factor receptors, but lose the careful control of normal proliferation. The loss of control may be caused by numerous factors, such as the overexpression of growth factors and/or receptors, and autonomous activation of biochemical pathways regulated by growth factors.

Some examples of receptors involved in tumorigenesis are the receptors for epidermal growth factor (EGFR), platelet-derived growth factor (PDGFR), insulin-like growth factor (IGFR), nerve growth factor (NGFR), and fibroblast growth factor (FGF).

Members of the epidermal growth factor (EGF) receptor family are particularly important growth factor receptor tyrosine kinases associated with tumorigenesis of epidermal cells. The first member of the EGF receptor family to be discovered was the glycoprotein having an apparent molecular weight of approximately 165 kD. This glycoprotein, which was described by Mendelsohn *et al.* in U.S. Patent No. 4,943,533, is known as the EGF receptor (EGFR) and also as human EGF receptor-1 (HER1).

The EGFR is overexpressed on many types of epidermoid tumor cells. EGF and transforming growth factor alpha (TGF-alpha) are two known ligands of EGFR. Examples of tumors that express EGF receptors include glioblastomas, as well as cancers of the lung, breast, head and neck, and bladder. The amplification and/or overexpression of the EGF receptors on the membranes of tumor cells is associated with a poor prognosis.

Treatments of cancer traditionally include chemotherapy or radiation therapy. Some examples of chemotherapeutic agents include doxorubicin, cisplatin, and taxol. The radiation can be either from an external beam or from a source placed inside a patient, i.e., brachytherapy.

Another type of treatment includes antagonists of growth factors or growth factor receptors involved in the proliferation of cells. Such antagonists neutralize the activity of the growth factor or receptor, and inhibit the growth of tumors that express the receptor.

For example, U.S. Patent No. 4,943,533 describes a murine monoclonal antibody called 225 that binds to the EGF receptor. The patent is assigned to the University of California and licensed exclusively to ImClone Systems Incorporated. The 225 antibody is able to inhibit the growth of cultured EGFR-expressing tumor lines as well as the growth of these tumors *in vivo* when grown as xenografts in nude mice. See Masui *et al.,* Cancer Res. 44, 5592-5598 (1986).

A disadvantage of using murine monoclonal antibodies in human therapy is the possibility of a human anti-mouse antibody (HAMA) response due to the presence of mouse Ig sequences. This disadvantage can be minimized by replacing the entire constant region of a murine (or other non-human mammalian) antibody with that of a human constant region. Replacement of the constant regions of a murine antibody with human sequences is usually referred to as chimerization.

The chimerization process can be made even more effective by also replacing the framework variable regions of a murine antibody with the corresponding human sequences. The framework variable regions are the variable regions of an antibody other than the hypervariable regions. The hypervariable regions are also known as the complementarity-determining regions (CDRs).

The replacement of the constant regions and framework variable regions with human sequences is usually referred to as humanization. The humanized antibody is less immunogenic (i.e. elicits less of a HAMA response) as more murine sequences are replaced by human sequences. Unfortunately, both the cost and effort increase as more regions of a murine antibodies are replaced by human sequences.

The replacement of non-human constant regions with human constant regions is not expected to affect the activity of an antibody. For example, Prewett et al. reported the inhibition of tumor progression of well-established prostate tumor xenografts in mice with a chimeric form of the anti-EGFR 225 monoclonal antibody discussed above. The chimeric form is called c225. Journal of Immunotherapy 19, 419-427 (1997).

Another approach to reducing the immunogenicity of antibodies is the use of antibody fragments. For example, an article by Aboud-Pirak et al., Journal of the National Cancer Institute 80, 1605-1611 (1988), compares the anti-tumor effect of an anti-EGF receptor antibody called 108.4 with fragments of the antibody. The tumor model was based on KB cells as xenografts in nude mice. KB cells are derived from human oral epidermoid carcinomas, and express elevated levels of EGF receptors.

Aboud-Pirak *et al.* found that both the antibody and the bivalent F(ab')₂ fragment retarded tumor growth in vivo, although the F(ab')₂ fragment was less efficient. The monovalent Fab fragment of the antibody, whose ability to bind the cell-associated receptor was conserved, did not, however, retard tumor growth.

Attempts have also been made to improve cancer treatments by combining some of the techniques mentioned above. For example, Baselga et al. reported anti-tumor effects of the chemotherapeutic agent doxorubicin with anti-EGFR monoclonal antibodies in the Journal of the National Cancer Institute 85, 1327-1333 (1993).

Others have attempted to enhance the sensitivity of cancer cells to radiation by combining the radiation with adjuvants. For example, Bonnen, U.S. Patent 4,846,782, reported increased sensitivity of human cancers to radiation when the radiation was combined with interferon. Snelling et al. reported a minor improvement in the radiation treatment of patients with astrocytomas with anaplastic foci when the radiation was combined with an anti-EGFR monoclonal antibody radiolabeled with iodine-125 in a phase II clinical trial. See Hybridoma 14, 111-114 (1995).

Similarly, Balaban et al. reported the ability of anti-EGFR monoclonal antibodies to sensitize human squamous carcinoma xenografts in mice to radiation when the radiation treatment was preceded by administration of an anti-EGFR antibody called LA22. See Biochimica et Biophysica Acta 1314. 147-136 (1996). Saleh et al. also reported better tumor control *in vitro* and in mice when radiation therapy was augmented with anti-EGFR monoclonal antibodies. Saleh et al. concluded that: "Further studies...may lead to a novel combined modality RT/Mab therapy." See abstract 4197 in the proceedings of the American Association for Cancer Researchs 7, 612 (1996).

Despite the above described treatments to fight cancer, none have been directed specifically at treating tumors refractory to conventional chemotherapy and radiation. Refractory tumors lead to rapid disease progression, usually with a poor prognosis. Currently there is little that can be done for patients with tumors refractory to conventional cancer treatment.

Based on the foregoing, there is a need for an improved method of treating refractory tumors in humans.

### SUMMARY OF THE INVENTION

This, and other objectives as will be apparent to those having ordinary skill in the art, have been achieved by providing a method of inhibiting the growth of refractory tumors that are stimulated by a ligand of epidermal growth factor receptor (EGFR) in human patients. The method comprises treating the human patients with an effective amount of an EGFR/HER1 antagonist.

In another embodiment, the method of the present invention comprises treating human patients with a combination of an effective amount of an EGFR/HER1 antagonist and a chemotherapeutic agent.

In yet another embodiment, the method of the present invention comprises treating human patients with a combination of an effective amount of an EGFR/HER1 antagonist and radiation.

### DETAILED DESCRIPTION OF THE INVENTION

The present invention provides an improved method for treating refractory tumors, particularly refractory malignant tumors, in human patients who have refractory cancer.

### Refractory Tumors

Refractory tumors include tumors that fail or are resistant to treatment with chemotherapeutic agents alone, radiation alone or combinations thereof. For the purposes of this specification, refractory tumors also encompass tumors that appear to be inhibited by treatment with chemotherapeutic agents and/or radiation but recur up to five years, sometimes up to ten years or longer after treatment is discontinued.

The types of refractory tumors that can be treated in accordance with the invention are any refractory tumors that are stimulated by a ligand of EGFR. Some examples of ligands that stimulate EGFR include EGF and TGF-alpha.

The EGFR family of receptors includes EGFR, which is also referred to in the literature as HER1. In this specification, EGFR refers to the specific member of the EGFR family of receptors called EGFR/HER1.

The refractory tumors treatable by the present invention are endogenous tumors native to human patients. These tumors are more difficult to treat than exogenous human tumor xenografts that were treated in animals. See, for example, Prewett et al., Journal of Immunotherapy 19, 419-427 (1997).

Some examples of refractory tumors include carcinomas, gliomas, sarcomas, adenocarcinomas, adenosarcomas and adenomas. Such tumors occur in virtually all parts of the human body, including every organ. The tumors may, for example, be present in the breast, heart, lung, small intestine, colon, spleen, kidney, bladder, head and neck, ovary, prostate, brain, pancreas, skin, bone, bone marrow, blood, thymus, uterus, testicles, cervix, and liver.

The tumors may express EGFR at normal levels or they may overexpress EGFR at levels, for example, that are at least 10, 100 or 1000 times normal levels. Some tumors that overexpress the EGFR include breast, lung, colon, kidney, bladder, head and neck, especially squamous cell carcinoma of the head and neck, ovary, prostate, and brain.

### EGFR/HER1 antagonists

The refractory tumors of the present invention can be treated with an EGFR/HER1 antagonist. For the purposes of this specification, an EGFR/HER1 antagonist is any substance that inhibits the stimulation of EGFR/HER1 by an EGFR/HER1 ligand. Such inhibition of stimulation inhibits the growth of cells that express EGFR/HER1.

The growth of refractory tumors is sufficiently inhibited in the patient to prevent or reduce the progression of the cancer (i.e. growth, invasiveness, metastasis, and/or recurrence). The EGFR antagonists of the present invention can be cytostatic or inhibit the growth of the refractory tumor. Preferably, the ERGR antagonist is cytolytic or destroys the tumor.

No particular mechanism of inhibition is implied as operating in the present invention. Nevertheless, EGFR tyrosine kinases are generally activated by means of phosphorylation events. Accordingly, phosphorylation assays are useful in predicting the antagonists useful in the present invention. Some useful assays for EGFR tyrosine kinase activity are described in Panek et al., Journal of Pharmacology and Experimental Therapeutics 283, 1433-1444 (1997) and in Batley et al., Life Sciences 62, 143-150 (1998). The description of these assays is incorporated herein by reference.

EGFR/HER1 antagonists include biological molecules or small molecules. Biological molecules include all lipids and polymers of monosaccharides, amino acids and nucleotides having a molecular weight greater than 450. Thus, biological molecules include, for example, oligosaccharides and polysaccharides; oligopeptides, polypeptides, peptides, and proteins; and oligonucleotides and polynucleotides. Oligonucleotides and polynucleotides include, for example, DNA and RNA.

Biological molecules further include derivatives of any of the molecules described above. For example, derivatives of biological molecules include lipid and glycosylation derivatives of oligopeptides, polypeptides, peptides and proteins. Derivatives of biological molecules further include lipid derivatives of oligosaccharides and polysaccharides, e.g. lipopolysaccharides. Most typically, biological molecules are antibodies, or functional equivalents of antibodies.

Functional equivalents of antibodies have binding characteristics comparable to those of antibodies, and inhibit the growth of cells that express EGFR. Such functional equivalents include, for example, chimerized, humanized and single chain antibodies as well as fragments thereof.

Functional equivalents of antibodies also include polypeptides with amino acid sequences substantially the same as the amino acid sequence of the variable or hyper-variable regions of the antibodies of the invention. An amino acid sequence that is substantially the same as another sequence, but that differs from the other sequence by means of one or more substitutions, additions, and/or deletions, is considered to be an equivalent sequence. Preferably, less than 50%, more preferably less than 25%, and still more preferably less than 10%, of the number of amino acid residues in a sequence are substituted for, added to, or deleted from the protein.

The functional equivalent of an antibody is preferably a chimerized or humanized antibody. A chimerized antibody comprises the variable region of a non-human antibody and the constant region of a human antibody. A humanized antibody comprises the hypervariable region (CDRs) of a non-human antibody. The variable region other than the hypervariable region, e.g. the framework variable region, and the constant region of a humanized antibody are those of a human antibody.

For the purposes of this application, suitable variable and hypervariable regions of non-human antibodies may be derived from antibodies produced by any non-human mammal in which monoclonal antibodies are made. Suitable examples of mammals other than humans include, for example, rabbits, rats, mice, horses, goats, or primates. Mice are preferred.

Functional equivalents further include fragments of antibodies that have binding characteristics that are the same as, or are comparable to, those of the whole antibody. Suitable fragments of the antibody include any fragment that comprises a sufficient portion of the hypervariable (i.e. complementarity determining) region to bind specifically, and with sufficient affinity, to EGFR tyrosine kinase to inhibit growth of cells that express such receptors.

Such fragments may, for example, contain one or both Fab fragments or the F(ab')₂ fragment. Preferably the antibody fragments contain all six complementarity determining regions of the whole antibody, although functional fragments containing fewer than all of such regions, such as three, four or five CDRs, are also included.

The preferred fragments are single chain antibodies, or Fv fragments. Single chain antibodies are polypeptides that comprise at least the variable region of the heavy chain of the antibody linked to the variable region of the light chain, with or without an interconnecting linker. Thus, Fv fragment comprises the entire antibody combining site. These chains may be produced in bacteria or in eukaryotic cells.

The antibodies and functional equivalents may be members of any class of immunoglobulins, such as: IgG, IgM, IgA, IgD, or IgE, and the subclasses thereof. The preferred antibodies are members of the IgGI subclass. The functional equivalents may also be equivalents of combinations of any of the above classes and subclasses.

Antibodies may be made from the desired receptor by methods that are well known in the art. The receptors are either commercially available, or can be isolated by well known methods. For example, methods for isolating and purifying EGFR are found in Spada, U.S. Patent 5,646,153 starting at column 41, line 55. The method for isolating and purifying EGFR described in the Spada patent is incorporated herein by reference.

Methods for making monoclonal antibodies include the immunological method described by Kohler and Milstein in Nature 256, 495-497 (1975) and by Campbell in "Monoclonal Antibody Technology, The Production and Characterization of Rodent and Human Hybridomas" in Burdon et al., Eds, Laboratory Techniques in Biochemistry and Molecular Biology, Volume 13, Elsevier Science Publishers, Amsterdam (1985). The recombinant DNA method described by Huse et al. in Science 246, 1275-1281 (1989) is also suitable.

Briefly, in order to produce monoclonal antibodies, a host mammal is inoculated with a receptor or a fragment of a receptor, as described above, and then, optionally, boosted. In order to be useful, the receptor fragment must contain sufficient amino acid residues to define the epitope of the molecule being detected. If the fragment is too short to be immunogenic, it may be conjugated to a carrier molecule. Some suitable carrier molecules include keyhold limpet hemocyanin and bovine serum albumin. Conjugation may be carried out by methods known in the art. One such method is to combine a cysteine residue of the fragment with a cysteine residue on the carrier molecule.

Spleens are collected from the inoculated mammals a few days after the final boost. Cell suspensions from the spleens are fused with a tumor cell. The resulting hybridoma cells that express the antibodies are isolated, grown, and maintained in culture.

Suitable monoclonal antibodies as well as growth factor receptor tyrosine kinases for making them are also available from commercial sources, for example, from Upstate Biotechnology, Santa Cruz Biotechnology of Santa Cruz, California, Transduction Laboratories of Lexington, Kentucky, R&D Systems Inc of Minneapolis, Minnesota, and Dako Corporation of Carpinteria, California.

Methods for making chimeric and humanized antibodies are also known in the art. For example, methods for making chimeric antibodies include those described in U.S. patents by Boss (Celltech) and by Cabilly (Genentech). See U.S. Patent Nos. 4,816,397 and 4,816,567, respectively. Methods for making humanized antibodies are described, for example, in Winter, U.S. Patent No. 5,225,539.

The preferred method for the humanization of antibodies is called CDR-grafting. In CDR-grafting, the regions of the mouse antibody that are directly involved in binding to antigen, the complementarity determining region or CDRs, are grafted into human variable regions to create "reshaped human" variable regions. These fully humanized variable regions are then joined to human constant regions to create complete "fully humanized" .antibodies.

In order to create fully humanized antibodies that bind well to an antigen, it is advantageous to design the reshaped human variable regions carefully. The human variable regions into which the CDRs will be grafted should be carefully selected, and it is usually necessary to make a few amino acid changes at critical positions within the framework regions (FRs) of the human variable regions.

For example, the reshaped human variable regions may include up to ten amino acid changes in the FRs of the selected human light chain variable region, and as many as twelve amino acid changes in the FRs of the selected human heavy chain variable region. The DNA sequences coding for these reshaped human heavy and light chain variable region genes are joined to DNA sequences coding for the human heavy and light chain constant region genes, preferably γ1 and κ, respectively. The reshaped humanized antibody is then expressed in mammalian cells and its affinity for its target compared with that of the corresponding murine antibody and chimeric antibody.

Methods for selecting the residues of the humanized antibody to be substituted and for making the substitutions are well known in the art. See, for example, Co et al., Nature 351, 501-502 (1992); Queen et al., Proc. Natl. Acad. Sci..86, 10029-1003 (1989) and Rodrigues et al., Int. J. Cancer, Supplement 7, 45-50 (1992). A method for humanizing and reshaping the 225 anti-EGFR monoclonal antibody described by Goldstein et al. in PCT application WO 96/40210. This method can be adapted to humanizing and reshaping antibodies against other growth factor receptor tyrosine kinases.

Methods for making single chain antibodies are also known in the art. Some suitable examples include those described by Wels et al. in European patent application 502 812 and Int. J. Cancer 60, 137-144 (1995).

Other methods for producing the functional equivalents described above are disclosed in PCT Application WO 93/21319, European Patent Application 239 400, PCT Application WO 89/09622, European Patent Application 338 745, U.S. Patent 5,658,570, U.S. Patent 5,693,780, and European Patent Application EP 332 424.

Preferred EGFR antibodies are the chimerized, humanized, and single chain antibodies derived from a murine antibody called 225, which is described in U.S. Patent No. 4,943,533. The patent is assigned to the University of California and licensed exclusively to ImClone Systems Incorporated.

The 225 antibody is able to inhibit the growth of cultured EGFR/HER1-expressing tumor cells *in vitro* as well as *in vivo* when grown as xenografts in nude mice. See Masui et al., Cancer Res. 44, 5592-5598 (1986). More recently, a treatment regimen combining 225 plus doxorubicin or cisplatin exhibited therapeutic synergy against several well established human xenograft models in mice. Basalga et al., J. Natl. Cancer Inst. 85, 1327-1333 (1993).

In one embodiment of the present invention, human patients with refractory head and neck squamous cell carcinoma were treated with a combination of an EGFR/HER1 antagonist (chimeric anti-EGFR monoclonal antibody, C225) and cisplatin. These patients had failed prior treatment with radiation alone, chemotherapy alone or combinations thereof. The EGFR/HER1 antagonist inhibited the growth of refractory tumors.

The chimerized, humanized, and single chain antibodies derived from murine antibody 225 can be made from the 225 antibody, which is available from the ATCC. Alternatively, the various fragments needed to prepare the chimerized, humanized, and single chain 225 antibodies can be synthesized from the sequence provided in Wels et al. in Int. J. Cancer 60, 137-144 (1995). The chimerized 225 antibody (c225) can be made in accordance with the methods described above. Humanized 225 antibody can be prepared in accordance with the method described in example IV of PCT application WO 96/40210, which is incorporated herein by reference. Single chain 225 antibodies (Fv225) can be made in accordance with methods described by Wels et al. in Int. J. Cancer 60, 137-144 (1995) and in European patent application 502 812.

The sequences of the hypervariable (CDR) regions of the light and heavy chain are reproduced below. The amino acid sequence is indicated below the nucleotide sequence.

### HEAVY CHAIN HYPERVARIABLE REGIONS (VH):

### CDR1

AACTATGGTGTACAC (SEQ ID 1)
N Y G V H (SEQ ID 2)

### CDR2

GTGATATGGAGTGGTGGAAACACAGACTATAATACACCTTTCACATCC (SEQ ID 3)
V I W S G G N T D Y N T P F T S (SEQ ID 4)

### CDR3

GCCCTCACCTACTATGATTACGAGTTTGCTTAC (SEQ ID 5)
A L T Y Y D Y E F A Y (SEQ ID 6)

### LIGHT CHAIN HYPERVARIABLE REGIONS (VL):

### CDR1

AGGGCCAGTCAGAGTATTGGCACAAACATACAC (SEQ ID 7)
R A S Q S I G T N I H (SEQ ID 8)

### CDR2

GCTTCTGAGTCTATCTCT (SEQ ID 9)
A S E S I S (SEQ ID 10)

### CDR3

CAACAAAATAATAACTGGCCAACCACG (SEQ ID 11)
Q Q N N N W P T T (SEQ ID 12)

In addition to the biological molecules discussed above, the antagonists useful in the present invention may also be small molecules. Any molecule that is not a biological molecule is considered in this specification to be a small molecule. Some examples of small molecules include organic compounds, organometallic compounds, salts of organic and organometallic compounds, saccharides, amino acids, and nucleotides. Small molecules further include molecules that would otherwise be considered biological molecules, except their molecular weight is not greater than 450. Thus, small molecules may be lipids, oligosaccharides, oligopeptides, and oligonucleotides, and their derivatives, having a molecular weight of 450 or less.

It is emphasized that small molecules can have any molecular weight. They are merely called small molecules because they typically have molecular weights less than 450. Small molecules include compounds that are found in nature as well as synthetic compounds. Preferably, the small molecules inhibit the growth of refractory tumor cells that express EGFR/HER1 tyrosine kinase.

Numerous small molecules have been described as being useful to inhibit EGFR. For example, Spada et al., U.S. Patent 5,656,655, discloses styryl substituted heteroaryl compounds that inhibit EGFR. The heteroaryl group is a monocyclic ring with one or two heteroatoms, or a bicyclic ring with 1 to about 4 heteroatoms, the compound being optionally substituted or polysubstituted. The compounds disclosed in U.S. Patent 5,656,655 are incorporated herein by reference.

Spada et al., U.S. Patent 5,646,153 discloses bis mono and/or bicyclic aryl heteroaryl, carbocyclic, and heterocarbocyclic compounds that inhibit EGFR. The compounds disclosed in U.S. Patent 5,646,153 are incorporated herein by reference.

Bridges et al., U.S. Patent 5,679,683 discloses tricyclic pyrimidine compounds that inhibit the EGFR. The compounds are fused heterocyclic pyrimidine derivatives described at column 3, line 35 to column 5, line 6. The description of these compounds at column 3, line 35 to column 5, line 6 is incorporated herein by reference.

Barker, U.S. Patent 5,616,582 discloses quinazoline derivatives that have receptor tyrosine kinase inhibitory activity. The compounds disclosed in U.S. Patent 5,616,582 are incorporated herein by reference.

Fry et al., Science 265, 1093-1095 (1994) discloses a compound having a structure that inhibits EGFR. The structure is shown in Figure 1. The compound shown in Figure 1 of the Fry et al. article is incorporated herein by reference.

Osherov et al., disclose tyrphostins that inhibit EGFR/HER1 and HER2. The compounds disclosed in the Osherov et al. article, and, in particular, those in Tables I, II, III, and REV are incorporated herein by reference.

Levitzki et al., U.S. Patent 5,196,446, discloses heteroarylethenediyl or heteroarylethenediylaryl compounds that inhibit EGFR. The compounds disclosed in U.S. Patent 5,196,446 from column 2, line 42 to column 3, line 40 are incorporated herein by reference.

Panek, et al., Journal of Pharmacology and Experimental Therapeutics 283, 1433-1444 (1997) disclose a compound identified as PD166285 that inhibits the EGFR, PDGFR, and FGFR families of receptors. PD166285 is identified as 6-(2,6-dichlorophenyl)-2-(4-(2-diethylaminoethoxy)phenylamino)-8-methyl-8H-pyrido(2,3-d)pyrimidin-7-one having the structure shown in Figure 1 on page 1436. The compound described in Figure 1 on page 1436 of the Panek et al. article is incorporated herein by reference.

### Administration of EGFR/HER1 antagonists

The present invention includes administering an effective amount of the EGFR/HER1 antagonist to human patients. Administering the EGFR/HER1 antagonists can be accomplished in a variety of ways including systemically by the parenteral and enteral routes. For example, EGFR/HER1 antagonists of the present invention can easily be administered intravenously (e.g., intravenous injection) which is a preferred route of delivery. Intravenous administration can be accomplished by contacting the EGFR/HER1 antagonists with a suitable pharmaceutical carrier (vehicle) or excipient as understood by those skilled in the art. The EGFR/HER1 antagonist may be administered with adjuvants, such as for example, BCG, immune system stimulators and chemotherapeutic agents.

EGFR/HER1 antagonists that are small molecule or biological drugs can be administered as described in Spada, U.S. Patent 5,646,153 at column 57, line 47 to column 59, line 67. This description of administering small molecules is incorporated herein by reference.

The EGFR/HER1 antagonists of the present invention significantly inhibit the growth of refractory tumor cells when administered to a human patient in an effective amount. As used herein, an effective amount is that amount effective to achieve the specified result of inhibiting the growth of the refractory tumor.

Preferably, the EGFR/HER1 antagonist is provided to the tumor in an amount which inhibits tumor growth without disrupting the growth of normal tissue. Most preferably, the EGFR/HER1 antagonist inhibits tumor growth without the serious side effects. Some serious side effects include bone marrow suppression, anemia and infection.

Optimal doses of EGFR/HER1 antagonists that are antibodies and functional equivalents of antibodies can be determined by physicians based on a number of parameters including, for example, age, sex, weight, severity of the condition being treated, the antibody being administered, and the route of administration. In general, a serum concentration of polypeptides and antibodies that permits saturation of the target receptor is desirable. For example, a concentration in excess of approximately 0.1 nM is normally sufficient. For example, a dose of 100 mg/m² ofC225 provides a serum concentration of approximately 20 nM for approximately eight days.

As a rough guideline, doses of antibodies may be given weekly in amounts of 10-300 mg/m². Equivalent doses of antibody fragments should be used at more frequent intervals in order to maintain a serum level in excess of the concentration that permits saturation of the receptors.

### Combination Therapy

In one preferred embodiment the refractory tumor can be treated with an effective amount of an EGFR/HER1 antagonist with chemotherapeutic agents, radiation or combinations thereof.

Examples of chemotherapeutic agents or chemotherapy include alkylating agents, for example, nitrogen mustards, ethyleneimine compounds, alkyl sulphonates and other compounds with an alkylating action such as nitrosoureas, cisplatin and dacarbazine; antimetabolites, for example, folic acid, purine or pyrimidine antagonists; mitotic inhibitors, for example, vinca alkaloids and derivatives of podophyllotoxin; cytotoxic antibiotics and camptothecin derivatives.

Camptothecin derivatives include, for example camptothecin, 7-ethyl camptothecin, 10-hydroxy-7-ethyl-camptothecin (SN38), 9-amino camptothecin, 10,1-methylenedioxy- camptothecin (MDCPT) and topotecan. Such camptothecin derivatives also include lactone stable formulations of 7-ethyl-camptothecin disclosed in U.S. Patent No. 5,604,233, the entire disclosure is incorporated herein by reference.

The present invention encompasses highly lipophilic camptothecin derivatives such as, for example, 10,11-methylenodioxy-camptothecin, 10,11-ethylenedioxy-camptothecin, 9-ethyl-camptothecin, 7-ethyl-10-hydroxy-camptothecin, 9-methyl-camptothecin, 9-chloro-10,11-methylenedioxy-camptothecin, 9-chloro camptothecin, 10-hydroxy-camptothecin, 9,10-dichloro camptothecin, 10-bromo-camptothecin, 10-chloro- camptothecin, 9-fluoro-camptothecin, 10-methyl-camptothecin, 10-fluoro-camptothecin, 9-methoxy-camptothecin, 9-chloro-7-ethyl-camptothecin and 11-fluoro-camptothecin. Such highly lipophilic camptothecin derivatives are disclosed in U.S. Patent No. 5,880,133, the entire disclosure is incorporated herein by reference.

Water soluble camptothecin derivatives include, for example, the water soluble analog of camptothecin known as CPT-11, 11-hydroxy-7-alkoxy-camptothecin, 11-hydroxy-7-methoxy camptothecin (11,7-HMCPT) and 11-hydroxy-7-ethyl camptothecin (11,7-HECPT), 7-dimethylaminomethylene-10,11-methylenedioxy-20(R,S)-camptothecin, 7-dimethylaminomethylene-10,11 - methylenedioxy-20(S)-camptothecin, 7-dimethylaminomethylene-10,11-ethylenedioxy-20(R,S)-camptothecin, and 7-morpholinomethylene- 10, 11 - ethylenedioxy-20(S)-camptothecin. Such water soluble camptothecin derivatives are disclosed in U.S. Patent Nos. 5,559,235 and 5,468,754, the entire disclosures are incorporated herein by reference.

Preferred chemotherapeutic agents or chemotherapy include amifostine (ethyol), cisplatin, dacarbazine (DTIC), dactinomycin, mechlorethamine (nitrogen mustard), streptozocin, cyclophosphamide, carmustine (BCNU), lomustine (CCNU), doxorubicin (adriamycin), doxorubicin lipo (doxil), gemcitabine (gemzar), daunorubicin, daunorubicin lipo (daunoxome), procarbazine, mitomycin, cytarabine, etoposide, methotrexate, 5-fluorouracil, vinblastine, vincristine, bleomycin, paclitaxel (taxol), docetaxel (taxotere), aldesleukin, asparaginase, busulfan, carboplatin, cladribine, camptothecin, CPT-11, 10-hydroxy-7-ethyl-camptothecin (SN38), dacarbazine, floxuridine, fludarabine, hydroxyurea, ifosfamide, idarubicin, mesna, interferon alpha, interferon beta, irinotecan, mitoxantrone, topotecan, leuprolide, megestrol, melphalan, mercaptopurine, plicamycin, mitotane, pegaspargase, pentostatin, pipobroman, plicamycin, streptozocin, tamoxifen, teniposide, testolactone, thioguanine, thiotepa, uracil mustard, vinorelbine, chlorambucil and combinations thereof.

Administering chemotherapeutic agents can be accomplished in a variety of ways including systemically by the parenteral and enteral routes. Preferably, the chemotherapeutic agent is administered intravenously by contacting the chemotherapeutic agent with a suitable pharmaceutical carrier (vehicle) or excipient as understood by those skilled in the art. The dose of chemotherapeutic agent depends on numerous factors as is well known in the art. Such factors include age, sex, weight, severity of the condition being treated, the agent being administered, and the route of administration. For example, cisplatin may conveniently be administered at a dose of about 100 mg/m². It should be emphasized, however, that the invention is not limited to any particular dose.

In yet another embodiment the refractory tumor can be treated with an effective amount of an EGFR/HER1 antagonist in combination with radiation. The source of radiation can be either external or internal to the patient being treated. When the source is external to the patient, the therapy is known as external beam radiation therapy (EBRT).
When the source of radiation is internal to the patient, the treatment is called brachytherapy (BT).

The radiation is administered in accordance with well known standard techniques with standard equipment manufactured for this purpose, such as AECL Theratron and Varian Clinac. The dose of radiation depends on numerous factors as is well known in the art. Such factors include the organ being treated, the healthy organs in the path of the radiation that might inadvertently be adversely affected, the tolerance of the patient for radiation therapy, and the area of the body in need of treatment. The dose will typically be between 1 and 100 Gy, and more particularly between 2 and 80 Gy. Some doses that have been reported include 35 Gy to the spinal cord, 15 Gy to the kidneys, 20 Gy to the liver, and 65-80 Gy to the prostate. It should be emphasized, however, that the invention is not limited to any particular dose. The dose will be determined by the treating physician in accordance with the particular factors in a given situation, including the factors mentioned above.

The distance between the source of the external radiation and the point of entry into the patient may be any distance that represents an acceptable balance between killing target cells and minimizing side effects. Typically, the source of the external radiation is between 70 and 100 cm from the point of entry into the patient.

Brachytherapy is generally carried out by placing the source of radiation in the patient. Typically, the source of radiation is placed approximately 0-3 cm from the tissue being treated. Known techniques include interstitial, intercavitary, and surface brachytherapy. The radioactive seeds can be implanted permanently or temporarily. Some typical radioactive atoms that have been used in permanent implants include iodine-125 and radon. Some typical radioactive atoms that have been used in temporary implants include radium, cesium-137, and iridium-192. Some additional radioactive atoms that have been used in brachytherapy include americium-241 and gold-198.

The dose of radiation for brachytherapy can be the same as that mentioned above for external beam radiation therapy. In addition to the factors mentioned above for determining the dose of external beam radiation therapy, the nature of the radioactive atom used is also taken into account in determining the dose of brachytherapy.

In the preferred embodiment, there is synergy when refractory tumors in human patients are treated with the EGFR/HER1 antagonist and chemotherapeutic agents or radiation or combinations thereof. In other words, the inhibition of tumor growth by the EGFR/HER1 antagonist is enhanced when combined with chemotherapeutic agents or radiation or combinations thereof. Synergy may be shown, for example, by greater inhibition of refractory tumor growth with combined treatment than would be expected from treatment with either the EGFR/HER1 antagonist, chemotherapeutic agent or radiation alone. Preferably, synergy is demonstrated by remission of the cancer where remission is not expected from treatment with EGFR/HER1 antagonist, chemotherapeutic agent or radiation alone.

The EGFR/HER1 antagonist is administered before, during, or after commencing chemotherapeutic agent or radiation therapy, as well as any combination thereof, i.e. before and during, before and after, during and after, or before, during, and after commencing the chemotherapeutic agent and/or radiation therapy. For example when the EGFR/HER1 antagonist is an antibody, it is typically administered between 1 and 30 days, preferably between 3 and 20 days, more preferably between 5 and 12 days before commencing radiation therapy and/or chemotherapeutic agents.

### Example 1. Clinical Trial

In a clinical trial, human patients with refractory head and neck squamous cell carcinoma were treated with a combination of an EGFR/HER1 antagonist (chimeric anti-EGFR monoclonal antibody, C225) and cisplatin. The patients received weekly infusions of C225 at loading/maintenance doses of 100/100, 400/250, or 500/250 mg/m² in combination with 100 mg/m² of cisplatin every three weeks. Tumor samples were obtained at baseline, 24 hours after the initial infusion and 24 hours before the third infusion to assess tumor EGFR saturation and function. Tumor EGFR saturation was assessed by immunohistochemistry (IHC) using M225 (murine counterpart of C225) as primary antibody and antimouse IgG as secondary antibody to detect unoccupied EGFR. The EGFR function was assessed by IHC using an antibody specific for activated EGFR (Transduction Labs) and measurement of EGFR tyrosine kinase activity on tumor lysates after clearing the C225-EGFR complexes. A dose dependent increase in receptor saturation was noted with greater than 70% receptor saturation through 500/250 mg/m² dose levels. Similarly, a significant reduction of EGFR-tyrosine kinase activity has been noted with no detectable activity in 67% of the patients at doses of 100/100 mg/m², suggesting functional saturation. Adverse events were fever, allergic reactions, and skin toxicity manifested as follicular rash or nail bed changes, which fully resolved after cessation of treatment. In seven evaluable patients there was one minimum, five partial, and one complete response as determined by physical exam and laboratory values.
Complete response was observed in one patient who had prior cisplatin treatment. Partial response was observed in five patients, four had prior chemotherapy, one had prior radiation treatment. Minimum response was observed in one patient with prior radiation treatment. The results are shown in the table, wherein CR means complete response, PR means partial response, and MR means minimum response.

**TABLE 1**

| Clinical Trial | | |
|---|---|---|
| Patient | Prior Treatment | Overall Response |
| 1 | Cisplatin | CR |
| 2 | Ad p53 | PR |
| 3 | Cisplatin | PR |
| 4 | Cisplatin | PR |
| 5 | Radiation alone | PR |
| 6 | Chemotherapy | PR |
| 7 | Radiation alone | MR |

### Example 2. Clinical Trial

In a clinical trial, one human patients with refractory colon cancer was treated with a combination of an EGFR/HER1 antagonist (chimeric anti-EGFR monoclonal antibody, C225) and CPT-11. The patient received weekly infusions of C225 at a loading dose of 400 mg/m² in combination with 125 mg/m² of CPT-11. Maintenance doses of 250 mg/m² C225 in combination with 69-125mg/m² of CPT-11 were administered on a weekly basis. Clinically, the patient had a complete response. The dosing schedule is summarized in Table 2 below.

**TABLE 2**

| Clinical Trial | | | |
|---|---|---|---|
| **C225/CPT-11** **weekly dose in mg/m²** | **C225/CPT-11** **(Actual dose in mg)** | **C225 Infusion Time (minutes)** | **CPT-11 Infusion Time (minutes)** |
| 400/125 | 576/180 | 120 | 90 |
| 250/125 | 360/180 | 60 | 90 |
| 250/CPT-11 Held | 360/0 | 60 | N/A |
| 250/94 | 360/135 | 50 | 75 |
| 250/69 | 360/100 | 60 | 85 |
| 250/69 | 360/100 | 60 | 75 |

## Claims

1. An effective amount of an EGFR/HER1 antagonist for use in human patients in a method of inhibiting the growth of refractory tumors that are stimulated by a ligand of epidermal growth factor receptor (EGFR).

2. The EGFR/HERI antagonist according to claim 1 wherein the antagonist is a monoclonal antibody specific for EGFRHER1 or a fragment that comprises the hypervariable region thereof.

3. The EGFR/HERI antagonist according to claim 2 wherein the monoclonal antibody is chimerized or humanized.

4. The EGFR/HERI antagonist according to claim 1 wherein the antagonist is a small molecule that binds specifically with EGFR/HER1.

5. The EGFR/HERI antagonist according to claim 4 wherein the small molecule inhibits EGFR/HER1 phosphorylation.

6. The EGFR/HERI antagonist according to claim 2 wherein the monoclonal antibody inhibits EGFR/HER1 phosphorylation.

7. The EGFR/HERI antagonist according to claim 1 wherein the refractory tumor has been treated with radiation or chemotherapy and combinations thereof.

8. The EGFR/HERI antagonist according to claim 1 wherein the tumors are tumors of the breast, heart, lung, small intestine, colon, spleen, kidney, bladder, head and neck, ovary, prostate, brain, pancreas, skin, bone, bone marrow, blood, thymus, uterus, testicles, cervix, and liver.

9. The EGFR/HERI antagonist according to claim 1 wherein the tumors are squamous cell carcinomas.

10. An effective amount of a combination of EGFR/HERI antagonist and radiation for use in human patients in a method of inhibiting the growth of refractory tumors that are stimulated by a ligand of epidermal growth factor receptor (EGFR).

11. The combination according to claim 10 wherein the antagonist is administered before radiation.

12. The combination according to claim 10 wherein the antagonist is administered during radiation.

13. The combination according to claim 10 wherein the antagonist is administered after the radiation.

14. The combination according to claim 10 wherein the antagonist is administered before and during radiation.

15. The combination according to claim 10 wherein the antagonist is administered during and after radiation.

16. The combination according to claim 10 wherein the antagonist is administered before and after radiation.

17. The combination according to claim 10 wherein the antagonist is administered before, during, and after radiation.

18. The combination according to claim 10 wherein the source of the radiation is external to the human patient.

19. The combination according to claim 10 wherein the source of radiation is internal to the human patient.

20. The combination according to claim 10 wherein the antagonist is a monoclonal antibody.

21. The combination according to claim 10 wherein the tumors are tumors of the breast, heart, lung, small intestine, colon, spleen, kidney, bladder, head and neck, ovary, prostate, brain, pancreas, skin, bone, bone marrow, blood, thymus, uterus, testicles, cervix, and liver.

22. A combination of an effective amount of an EGFR/HERI antagonist and a chemotherapeutic agent for use in human patients in a method of inhibiting the growth of refractory tumors that are stimulated by a ligand of epidermal growth factor receptor (EGFR).

23. The combination according to claim 22 wherein the antagonist is administered before treatment with the chemotherapeutic agent.

24. The combination according to claim 22 wherein the antagonist is administered during treatment with the chemotherapeutic agent.

25. The combination according to claim 22 wherein the antagonist is administered after the treatment with the chemotherapeutic agent.

26. The combination according to claim 22 wherein the antagonist is administered before treatment with the chemotherapeutic agent.

27. The combination according to claim 22 wherein the antagonist is administered during and after treatment with the chemotherapeutic agent.

28. The combination according to claim 22 wherein the antagonist is administered before and after treatment with the chemotherapeutic agent.

29. A method according to claim 22 wherein the antagonist is administered before, during, and after treatment with the chemotherapeutic agent.

30. The combination according to claim 22 wherein the chemotherapeutic agent is selected from the group consisting of amifostine, cisplatin, dacarbazine, dactinomycin, mechlorethamine, streptozocin, cyclophosphamide, carmustine, lomustine, doxorubicin, doxorubicin lipo, gemcitabine, daunorubicin, procarbazine, mitomycin, cytarabine, etoposide, methotrexate, 5-fluorouracil, vinblastine, vincristine, bleomycin, paclitaxel, docetaxel, aldesleukin, asparaginase, busulfan, carboplatin, cladribine, camptothecin, CPT-11, 10-hydroxy-7-ethyl-camptothecin (SN38), dacarbazine, floxuridine, fludarabine, hydroxyurea, ifosfamide, idarubicin, mesna, interferon alpha, interferon beta, irinotecan, mitoxantrone, topotecan, leuprolide, megestrol, melphalan, mercaptopurinc, plicamycin, mitotane, pegaspargase, pentostatin, pipobroman, plicamycin, streptozocin, tamoxifen, teniposide, testolactone, thioguanine, thiotepa, uracil mustard, vinorelbine, chlorambucil and combinations thereof.

31. The combination according to claim 22 wherein the chemotherapeutic agent is selected from the group consisting of cisplatin, doxorubicin, paclitaxel, CPT-11, topotecan and combinations thereof.

32. The combination according to claim 22 wherein the tumors are tumors of the breast, heart, lung, small intestine, colon, spleen, kidney, bladder, head and neck, ovary, prostate, brain, pancreas, skin, bone, bone marrow, blood, thymus, uterus, testicles, cervix, and liver.

33. The combination according to claim 22 wherein the antagonist is a monoclonal antibody.
